# FASCICULE DE BREVET EUROPEEN

(11) **EP 3 482 789 B1**
(45) Date de publication et mention de la délivrance du brevet: **29.04.2020**
(21) Numéro de dépôt: 18202372.1
(22) Date de dépôt: 24.10.2018
(51) Int. Cl.: A61M 16/00

(54) **APPAREIL D'ASSISTANCE RESPIRATOIRE À CIRCUIT DE GAZ AMÉLIORÉ**
BEATMUNGSGERÄT MIT VERBESSEREM GASKREIS
BREATHING APPARATUS WITH IMPROVED GAS CIRCUIT

(30) Priorité: 10.11.2017 FR 1760569
(43) Date de publication de la demande: 15.05.2019
(73) Titulaire: Air Liquide Medical Systems, 92160 Antony (FR)
(72) Inventeur: DAVOINE, Romain, 92160 ANTONY (FR)
(74) Mandataire: Pittis, Olivier

(56) Documents cités:
- EP-A1- 2 543 315
- WO-A1-2012/139681
- US-A1- 2016 184 539

## Description

L'invention concerne un appareil d'assistance respiratoire à micro-soufflante motorisée et chemin d'air amélioré.

Afin d'assister certains patients dans leur fonction respiratoire, on utilise des appareils d'assistance respiratoire ou de ventilation assistée, aussi appelés « ventilateurs » médicaux, qui délivrent un gaz respiratoire à un débit non nul et/ou à une pression supérieure à la pression atmosphérique (i.e. > 1 atm), typiquement de l'air (env. 21% d'O2 en vol.) ou de l'air enrichi en oxygène (>21 % d'O2 en vol.).

Pour ce faire, on utilise des appareils d'assistance respiratoire mettant en œuvre une micro-soufflante, parfois simplement appelée « soufflante », «compresseur» ou «turbine », servant à aspirer l'air ambiant et à le délivrer à une pression donnée aux patients. Les documents EP-A-2165078, EP-A-2102504 et WO-A-2012/139681 décrivent de tels ventilateurs à micro-soufflante.

Classiquement, l'aspiration de l'air par la micro-soufflante se fait grâce à une (ou plusieurs) roue à ailettes agencée sur un arbre-moteur rotatif entraîné en rotation par un moteur électrique. La roue de la micro-soufflante est généralement agencée dans un compartiment interne aménagé dans une volute définissant un volume creux.

La micro-soufflante est quant à elle agencée dans la carcasse ou coque de l'appareil qui sert non seulement à la maintenir et à la protéger contre les chocs éventuels, par exemple en cas de chute de l'appareil sur le sol, mais qui assure aussi un rôle d'atténuation acoustique du bruit généré par celle-ci. En effet, les ventilateurs destinés à traiter des pathologies du sommeil, telle l'apnée du sommeil, doivent être particulièrement silencieux pour ne pas gêner le patient ou son conjoint, pendant son/leur sommeil.

L'air circule dans le ventilateur, avant et après son passage dans la micro-soufflante, dans un ensemble pneumatique comprenant des conduits de gaz et des passages internes de l'appareil, lequel est appelé 'chemin d'air'.

Le chemin d'air raccorde fluidiquement des cavités cloisonnées (entrée du ventilateur, entrée/sortie de la micro-soufflante, sortie vers le patient...) et des moyens de mesures pneumatiques de pression et débit, c'est-à-dire des capteurs ou analogues.

En effet, il est indispensable de réaliser des mesures de pression et/ou de débit du gaz circulant dans le ventilateur pour assurer un pilotage efficace de la ventilation du patient traité et par ailleurs garantir sa sécurité en évitant notamment les barotraumatismes.

Actuellement, l'agencement et/ou le raccordement fluidique des capteurs ou analogues au sein du chemin d'air est problématique, en particulier du fait qu'il peut être composé de plusieurs pièces nécessitant une intégration complexe et un nombre important d'étapes de montage.

Le problème qui se pose est de proposer un appareil d'assistance respiratoire amélioré permettant un montage aisé des capteurs ou analogues au sein du chemin d'air tout en assurant des performances telles que l'étanchéité pneumatique.

La solution concerne alors un appareil d'assistance respiratoire, c'est-à-dire un ventilateur médical, comprenant un boitier contenant une micro-soufflante motorisée, caractérisé en ce que :
- le boitier est formé d'au moins une première et une seconde partie de boitier assemblées l'une à l'autre, la première partie de boitier comprenant un premier compartiment interne et un deuxième compartiment interne,
- la micro-soufflante est agencée dans le premier compartiment interne en étant prise en sandwich entre les première et seconde parties de boitier,
- la sortie de la micro-soufflante est en communication fluidique avec le deuxième compartiment interne,
- le deuxième compartiment interne comprend un orifice de sortie de gaz en communication fluidique avec un élément de conduit solidaire de la première partie de boitier, et
- l'élément de conduit comprend un passage de gaz et au moins une première, une seconde et une troisième tubulure de prise de pression solidaires de l'élément de conduit et en communication fluidique avec le passage de gaz.

Selon le cas, l'invention peut comprendre l'une ou plusieurs des caractéristiques techniques suivantes :
- un premier élément amortisseur en matériau souple est agencé sur la première partie de boitier, ledit premier élément amortisseur comprenant une expansion de paroi conformée pour épouser au moins une partie des contours extérieurs de l'élément de conduit et des première, seconde et troisième tubulures de prise de pression de manière à assurer une étanchéité fluidique entre eux.
- le premier élément amortisseur est pris en sandwich entre les première et seconde parties de boitier.
- l'expansion de paroi du premier élément amortisseur comprend une portion arquée de forme semi-cylindrique et au moins trois portions de conduits solidaires de la portion arquée et en communication fluidique avec l'intérieur de ladite portion arquée.
- les trois portions de conduits sont emmanchés autour des première, seconde et troisième tubulures de prise de pression, lorsque élément amortisseur est agencé sur la première partie de boitier.
- le premier élément amortisseur comprend une ouverture à carter, et la micro-soufflante comprend un moteur électrique agencé dans un carter, ledit carter de la micro-soufflante venant se loger dans l'ouverture à carter du premier élément amortisseur.
- un second élément amortisseur en matériau souple comprenant un passage interne est agencé au niveau de la sortie de la micro-soufflante et solidarisée à une cloison interne séparant le premier compartiment interne et le deuxième compartiment interne de manière à assurer une étanchéité fluidique entre le premier compartiment interne et le deuxième compartiment interne et une communication fluidique entre la sortie de la micro-soufflante et le deuxième compartiment interne.
- le second élément amortisseur est pris en sandwich entre la cloison interne de la première partie de boitier et le premier élément amortisseur.
- les premier et/ou second éléments amortisseurs sont en élastomère ou en thermoplastique élastomère, et/ou les première et seconde parties de boitier sont en polymère.
- la seconde partie de boitier comprend au moins trois orifices de prise de pression coopérant avec les au moins trois portions de conduits du premier élément amortisseur.
- la seconde partie de boitier comprend plusieurs capteurs de pression comprenant des prises de pression raccordées fluidiquement aux première, seconde et troisième tubulures de prise de pression de l'élément conduit de la première partie de boitier par l'intermédiaire des au moins trois portions de conduits du premier élément amortisseur.
- les capteurs de pression sont portés par au moins une carte électronique agencée dans la seconde partie de boitier.
- au moins un capteur de pression est un capteur de pression différentiel raccordé à deux tubulures de prise de pression desdites première, seconde et troisième tubulures de prise de pression de l'élément de conduit.
- un élément à perte de charge est agencé dans le passage de gaz entre deux tubulures de prise de pression desdites première, seconde et troisième tubulures de prise de pression de l'élément de conduit.

L'invention va maintenant être mieux comprise grâce à la description détaillée suivante, faite à titre illustratif mais non limitatif, en référence aux figures annexées parmi lesquelles :
- les Figures 1 à 3 schématisent -en vue éclatée- l'agencement d'une micro-soufflante au sein du boitier d'un appareil d'assistance respiratoire selon l'invention,
- la Figure 4 est une vue assemblée des éléments des Figures 1 et 2,
- les Figures 5 à 8 schématisent l'élément de conduit du boitier d'un appareil d'assistance respiratoire selon l'une des Figure 1 à 4, et
- la Figure 9 schématise l'élément à perte de charge agencé dans l'élément de conduit des Figures 1 à 8.

Un mode de réalisation de composants du boitier d'un appareil d'assistance respiratoire selon l'invention est illustré sur les Figures ci-jointes.

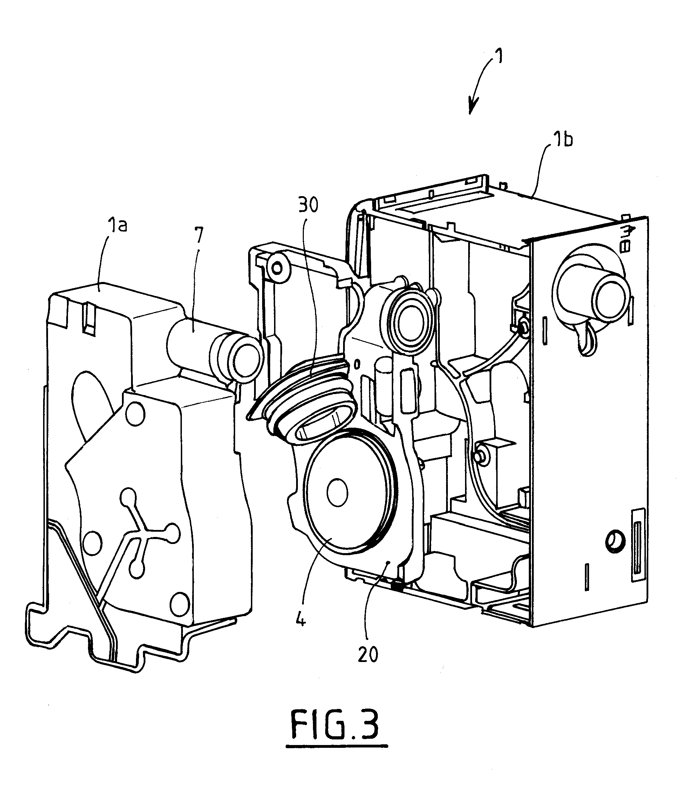

Il comprenant un boitier 1 rigide formé de deux demi-boitiers, c'est-à-dire d'une première 1a et d'une deuxième 1b parties de boitier assemblées l'une à l'autre comme illustré en Figure 3, et maintenues ensuite par emboitement (clipsage), vissage ou analogue. Les première 1a et seconde 1b parties de boitier sont réalisées en matériau thermoplastique, tel que ABS ou ABS-PC, et ont une épaisseur comprise entre 1 et 3 mm environ.

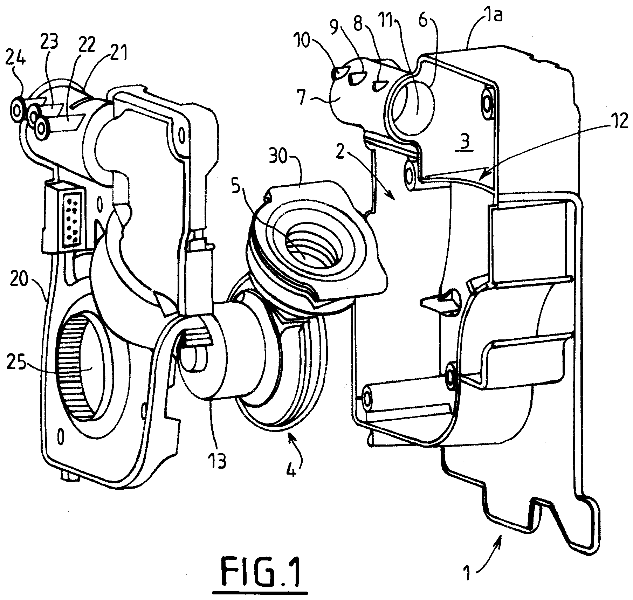

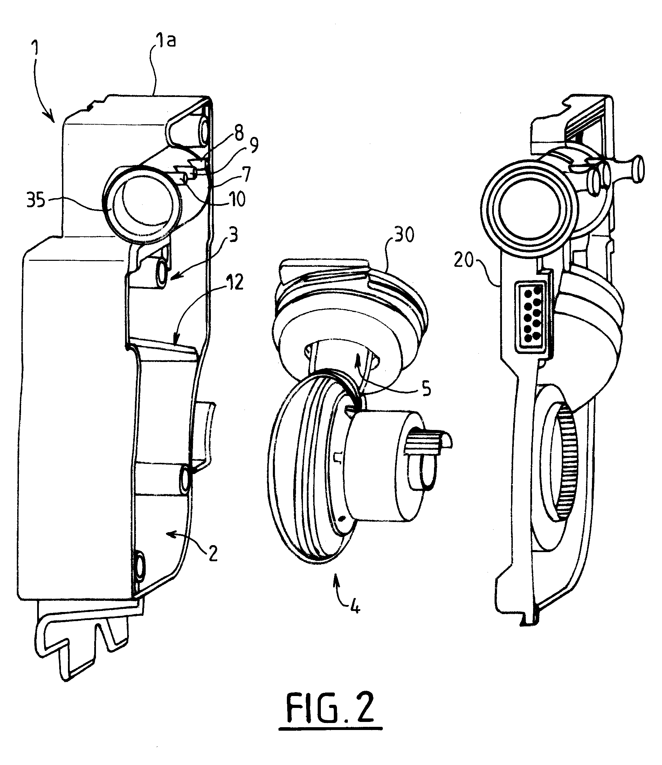

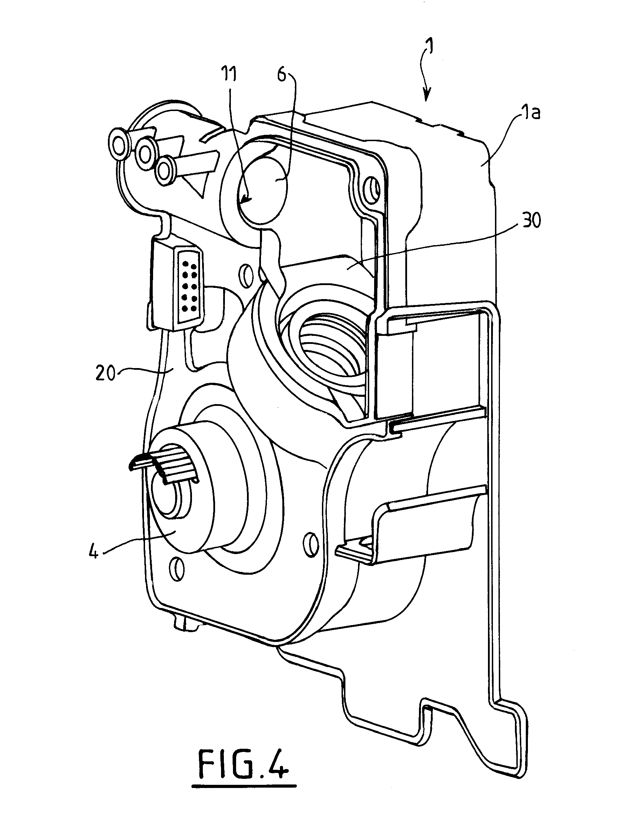

Une micro-soufflante 4 motorisée, c'est-à-dire à moteur électrique, est agencée dans un premier compartiment interne 2 de la première partie de boitier 1a en étant prise en sandwich entre les première et seconde parties 1a, 1b de boitier, comme illustré en Figures 1 à 4. La micro-soufflante 4 motorisée est classiquement alimentée en courant électrique par des câbles ou analogue servant à convoyer le courant électrique délivré par une source de courant électrique, par exemple le secteur électrique, une ou plusieurs batteries ou autres.

La sortie 5 de la micro-soufflante 4 délivrant le gaz sous pression est en communication fluidique avec un deuxième compartiment interne 3 aménagé dans la première partie de boitier 1a. Le premier compartiment interne 2 et le deuxième compartiment interne 3 sont séparés par une cloison interne 12 aménagée dans la première partie de boitier 1a.

Par ailleurs, le deuxième compartiment interne 3 comprend un orifice de sortie 6 de gaz qui est en communication fluidique avec le passage de gaz 11 d'un élément de conduit 7 solidaire de la première partie de boitier 1a. De préférence, l'élément de conduit 7 est formé d'une pièce, par exemple par moulage, avec le reste de la première partie de boitier 1a.

Cet élément de conduit 7 comprend aussi une première, une seconde et une troisième tubulure de prise de pression 8, 9, 10 qui sont solidaires dudit élément de conduit 7, de préférence formées d'une pièce, par exemple par moulage

Les première, seconde et troisième tubulures de prise de pression 8, 9,10 de l'élément de conduit 7 sont en communication fluidique avec le passage de gaz 11 de manière à pouvoir servir de sites de mesure de pression pour des capteurs de pression 50, 51, comme expliqué ci-après.

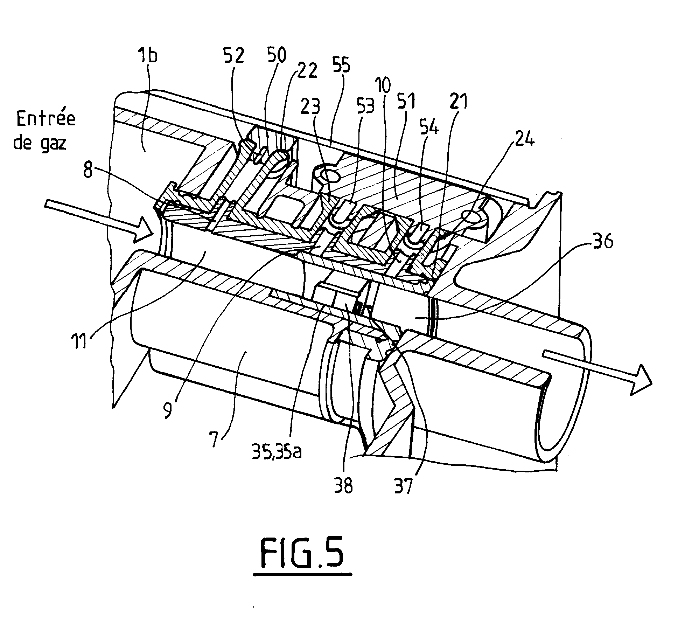

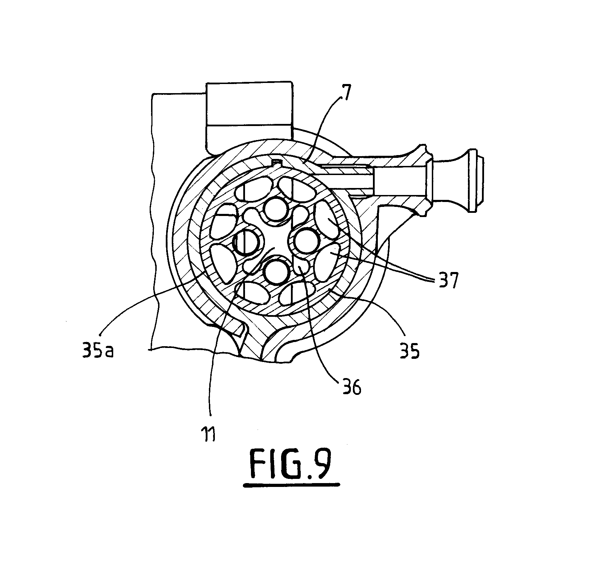

On voit par ailleurs qu'un élément à perte de charge 35 est agencé dans le passage de gaz 11 entre deux tubulures de prise de pression 9, 10 des première, seconde et troisième tubulures de prise de pression 8, 9,10 de l'élément de conduit 11 ; ceci est détaillé sur les Figures 5 à 9.

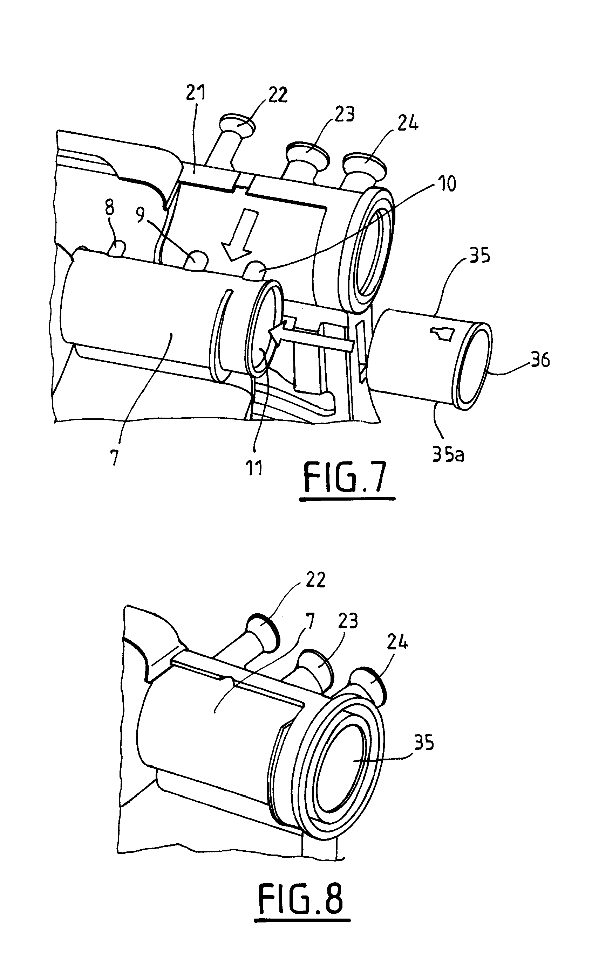

L'élément à perte de charge 35 est de forme générale tubulaire, i.e. de corps cylindrique 35a, comme illustré en Figure 7. Par exemple, il a une longueur de l'ordre de 10 à 20 mm, et un diamètre de l'ordre de 15 à 22 mm. Bien entendu, d'autres dimensionnements sont possibles.

Le corps cylindrique 35a de l'élément à perte de charge 35 comprend dans son lumen 36, c'est-à-dire dans le passage interne traversant son corps tubulaire, une portion 38 formée d'une pluralité de canaux 37 agencés axialement et en parallèle les uns des autres de sorte que le flux de gaz, i.e. d'air, puisse les traverser en perdant une partie de sa charge, i.e. de sa pression, de sorte d'obtenir un différentiel de pression (ΔP) entre l'amont et l'aval de ladite portion 38 formée de la pluralité de canaux 37. Ce différentiel de pression pourra être exploité par un capteur de pression différentiel 51 pour déterminer le débit du flux traversant l'élément de conduit 11, comme expliqué ci-dessous.

De plus ces canaux 37, préférentiellement en forme de nid d'abeilles, permettent au flux de gaz d'être redressé lors de son passage au travers de ceux-ci, limitant ainsi les perturbations aérauliques pouvant générer des bruits pneumatiques et acoustiques. Suivant le différentiel de pression devant être mesuré, l'épaisseur des parois constituant ces canaux est de l'ordre de 0,5 à 1 mm, et les diamètres moyens de ces canaux peuvent varier de 1 à 5 mm. La longueur de passage que constitue l'élément 37 peut par exemple être de l'ordre de 0,5 à 8 mm. Là encore, d'autres dimensionnements sont possibles.

Par ailleurs, un premier élément amortisseur 20 en matériau souple, par exemple en thermoplastique élastomère ou en élastomère de type silicone ou TPE, typiquement de 0,2 à 3 mm d'épaisseur, préférentiellement de dureté de l'ordre de 50 à 80 Shores A, est agencé sur la première partie de boitier 1a de manière à être pris en sandwich entre les première et seconde parties 1a, 1b du boitier 1, comme illustré en Figure 3, lorsque celles-ci sont assemblées l'une à l'autre. La micro-soufflante 4 est alors prise en sandwich entre le premier élément amortisseur 20 et la première partie de boitier 1a en étant agencée dans le premier compartiment interne 2. En fait, le carter 13 du moteur de la micro-soufflante 4 vient se loger dans une ouverture 25 agencée dans le premier élément amortisseur 20 de sorte d'être soutenu, c'est-à-dire maintenu en position suspendue, par ledit premier élément amortisseur 20.

Le premier élément amortisseur 20 comprend en outre une expansion de paroi 21-24 conformée pour épouser au moins une partie des contours extérieurs de l'élément de conduit 7 et des première, seconde et troisième tubulures 8, 9, 10 de prise de pression de manière à assurer une étanchéité fluidique entre eux.

L'expansion de paroi 21-24 comprend une portion arquée 21 de forme semi-cylindrique (ou quasi semi-cylindrique) et au moins trois portions de conduits 22, 23, 24 solidaires de la portion arquée 21 et en communication fluidique avec l'intérieur de ladite portion arquée 21. Ces trois portions de conduits 22, 23, 24 viennent s'emmancher étroitement, c'est-à-dire former des manchons, autour des première, seconde et troisième tubulures 8, 9,10 de prise de pression, ce qui permet d'obtenir l'étanchéité fluidique susmentionnée.

Comme visible en Figures 1 à 4, un second élément amortisseur 30, également en thermoplastique élastomère ou élastomère de type TPE ou silicone, est pris en sandwich entre la cloison interne 12 de la première partie de boitier 1a et le premier élément amortisseur 20 en matériau souple.

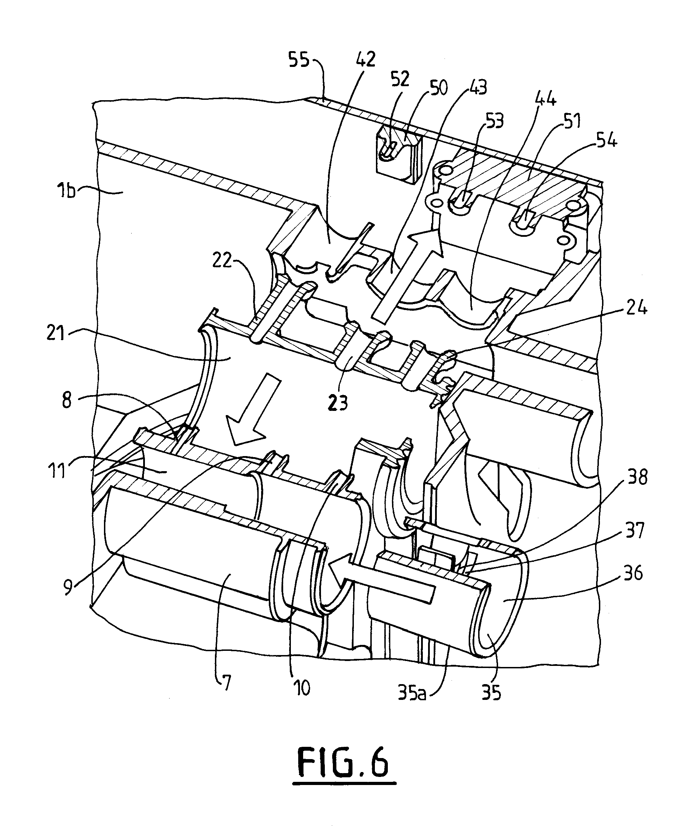

Comme illustré en Figures 6 et 7, la seconde partie de boitier 1b comprend au moins trois orifices de prise de pression 42, 43, 44 coopérant avec les au moins trois portions de conduits 22, 23, 24 du premier élément amortisseur 20. En effet, les trois portions de conduits 22, 23, 24 du premier élément amortisseur 20 viennent se loger, en étant positionné, dans les trois orifices de prise de pression 42, 43, 44, respectivement, de manière à assurer une continuité fluidique entre les première, seconde et troisième tubulures 8, 9, 10 de prise de pression et les prises de pression 52, 53, 54 de plusieurs capteurs de pression 50, 51 portés par une carte électronique 55 agencée dans la seconde partie de boitier 1b. L'étanchéité des tubulures extérieures 8, 9, 10, 52, 53, 54 est assurée par une déformation des éléments élastomères, de l'ordre de 0,2 à 0,5 mm, garantissant une étanchéité de 0,05 à 0,1 Bar.

Les première, seconde et troisième tubulures 8, 9, 10 de prise de pression de l'élément conduit 7 de la première partie de boitier 1a transmettent donc la ou les pressions régnant dans l'élément de conduit 11, notamment en amont et en aval de l'élément à perte de charge 35, par l'intermédiaire des trois portions de conduits 22, 23, 24 du premier élément amortisseur 20 et les trois orifices de prise de pression 42, 43, 44.

Les capteurs de pression 50, 51 comprennent ici un capteur de pression classique 50 permettant de déterminer la pression de gaz dans l'élément de conduit 11 et un capteur de pression différentiel 51 raccordé à deux tubulures de prise de pression 9, 10 de l'élément de conduit 7 de manière à déterminer un différentiel de pression du fait des valeurs de pression différentes régnant en amont et en aval de l'élément à perte de charge 35 agencé dans l'élément de conduit 11 entre les orifices d'entrée des deux portions de conduits 23, 24. Ce différentiel de pression permet ensuite à la carte électronique 55 de déterminer un débit de gaz.

De plus, la mesure de pression différentielle pour être « symétrique », c'est-à-dire identique dans les deux sens de mesure, lorsque le flux de gaz est poussé vers le patient ou lorsque le patient expire, l'élément 37 doit être positionné à égale distance des tubes 9 et 10.

La carte électronique 55, par exemple un circuit imprimé réalisé en matière époxy ou fibres de 1 à 3 mm d'épaisseur, comprend au moins un microprocesseur, de préférence un microcontrôleur, mettant en œuvre un ou des algorithmes. Les valeurs de pression et de débit peuvent ensuite être transmises par la carte électronique 55 à un afficheur digital, par exemple un écran alphanumérique ou analogue.

La carte électronique 55 assure en outre le pilotage de l'appareil et garantit la sécurité du patient.

L'appareil d'assistance respiratoire selon l'invention est destiné à délivrer un gaz respiratoire à des patients souffrant d'insuffisances respiratoires ou de tout autre trouble respiratoire affectant la fonction respiratoire de ces patients.

## Revendications

1. Appareil d'assistance respiratoire comprenant un boitier (1) contenant une micro-soufflante (4) motorisée, **caractérisé en ce que** :
- le boitier (1) est formé d'au moins une première et une seconde partie de boitier (1a, 1b) assemblées l'une à l'autre, la première partie de boitier (1a) comprenant un premier compartiment interne (2) et un deuxième compartiment interne (3),
- la micro-soufflante (4) est agencée dans le premier compartiment interne (2) en étant prise en sandwich entre les première et seconde parties de boitier (1a, 1b),
- la sortie (5) de la micro-soufflante (4) est en communication fluidique avec le deuxième compartiment interne (3),
- le deuxième compartiment interne (3) comprend un orifice de sortie (6) de gaz en communication fluidique avec un élément de conduit (7) solidaire de la première partie de boitier (1a), et
- l'élément de conduit (7) comprend un passage de gaz (11) et au moins une première, une seconde et une troisième tubulure de prise de pression (8,9,10) solidaires de l'élément de conduit (7) et en communication fluidique avec le passage de gaz (11).

2. Appareil selon la revendication précédente, **caractérisé en ce qu'**un premier élément amortisseur (20) en matériau souple est agencé sur la première partie de boitier (1a), ledit premier élément amortisseur (20) comprenant une expansion de paroi (21-24) conformée pour épouser au moins une partie des contours extérieurs de l'élément de conduit (7) et des première, seconde et troisième tubulures (8,9,10) de prise de pression de manière à assurer une étanchéité fluidique entre eux.

3. Appareil selon la revendication 2, **caractérisé en ce que** le premier élément amortisseur (20) est pris en sandwich entre les première et seconde parties de boitier (1a, 1b).

4. Appareil selon l'une des revendications 2 ou 3, **caractérisé en ce que** l'expansion de paroi (21-24) du premier élément amortisseur (20) comprend une portion arquée (21) de forme semi-cylindrique et au moins trois portions de conduits (22, 23, 24) solidaires de la portion arquée (21) et en communication fluidique avec l'intérieur de ladite portion arquée (21).

5. Appareil selon l'une des revendications 2 à 4, **caractérisé en ce que** les trois portions de conduits (22, 23, 24) sont emmanchés autour des première, seconde et troisième tubulures (8, 9,10) de prise de pression, lorsque élément amortisseur (20) est agencé sur la première partie de boitier (1a).

6. Appareil selon l'une des revendications 2 à 5, **caractérisé en ce que** le premier élément amortisseur (20) comprend une ouverture à carter (25), et la micro-soufflante (4) comprend un moteur électrique agencé dans un carter (13), ledit carter (13) de la micro-soufflante (4) venant se loger dans l'ouverture à carter (25) du premier élément amortisseur (20).

7. Appareil selon l'une des revendications précédentes, **caractérisé en ce que** qu'un second élément amortisseur (30) en matériau souple comprenant un passage interne (31) est agencé au niveau de la sortie (5) de la micro-soufflante (4) et solidarisée à une cloison interne (12) séparant le premier compartiment interne (2) et le deuxième compartiment interne (3) de manière à assurer une étanchéité fluidique entre le premier compartiment interne (2) et le deuxième compartiment interne (3) et une communication fluidique entre la sortie (5) de la micro-soufflante (4) et le deuxième compartiment interne (3).

8. Appareil selon la revendication 7, **caractérisé en ce que** le second élément amortisseur (30) est pris en sandwich entre la cloison interne (12) de la première partie de boitier (1a) et le premier élément amortisseur (20).

9. Appareil selon l'une des revendications 7 ou 8, **caractérisé en ce que** les premier et/ou second éléments amortisseurs (20, 30) sont en élastomère ou en thermoplastique élastomère, et/ou les première et seconde parties de boitier (1a, 1b) sont en polymère.

10. Appareil selon la revendication 2, **caractérisé en ce que** la seconde partie de boitier (1b) comprend au moins trois orifices de prise de pression (42, 43, 44) coopérant avec les au moins trois portions de conduits (22, 23, 24) du premier élément amortisseur (20).

11. Appareil selon la revendication 2, **caractérisé en ce que** la seconde partie de boitier (1b) comprend plusieurs capteurs de pression (50, 51) comprenant des prises de pression (52, 53, 54) raccordées fluidiquement aux première, seconde et troisième tubulures (8, 9,10) de prise de pression de l'élément conduit (7) de la première partie de boitier (1a) par l'intermédiaire des au moins trois portions de conduits (22, 23, 24) du premier élément amortisseur (20).

12. Appareil selon la revendication 11, **caractérisé en ce que** les capteurs de pression (50, 51) sont portés par au moins une carte électronique (55) agencée dans la seconde partie de boitier (1b).

13. Appareil selon l'une des revendications 11 ou 12, **caractérisé en ce que** :
- au moins un capteur de pression (50, 51) est un capteur de pression différentiel (51) raccordé à deux tubulures de prise de pression (9,10) desdites première, seconde et troisième tubulures de prise de pression (8, 9,10) de l'élément de conduit (7), et
- un élément à perte de charge (35) est agencé dans le passage de gaz (11) entre deux tubulures de prise de pression (9,10) desdites première, seconde et troisième tubulures de prise de pression (8, 9,10) de l'élément de conduit (7).

## Patentansprüche

1. Einrichtung zur Atemunterstützung mit einem Gehäuse (1), das ein motorisiertes Mikrogebläse (4) enthält, **dadurch gekennzeichnet, dass**:
- das Gehäuse (1) aus mindestens einem ersten und einem zweiten Gehäuseteil (1a, 1b) gebildet ist, die zusammengefügt sind, wobei der erste Gehäuseteil (1a) ein erstes Innenfach (2) und ein zweites Innenfach (3) umfasst,
- das Mikrogebläse (4) im ersten Innenfach (2) angeordnet ist, indem es zwischen dem ersten und zweiten Gehäuseteil (1a, 1b) eingeschlossen wird,
- der Ausgang (5) des Mikrogebläses (4) in Fluidkommunikation mit dem zweiten Innenfach (3) steht,
- das zweite Innenfach (3) eine Gasausgangsöffnung (6) in Fluidkommunikation mit einem Leitungselement (7) umfasst, das fest mit dem ersten Gehäuseteil (1a) verbunden ist, und
- das Leitungselement (7) einen Gasdurchlass (11) und mindestens einen ersten, einen zweiten und einen dritten Druckabgriffsstutzen (8, 9, 10) umfasst, die mit dem Leitungselement (7) fest verbunden sind und in Fluidkommunikation mit dem Gasdurchlass (11) stehen.

2. Einrichtung nach dem vorstehenden Anspruch, **dadurch gekennzeichnet, dass** ein erstes Dämpfungselement (20) aus flexiblem Material auf dem ersten Gehäuseteil (1a) angeordnet ist, wobei das erste Dämpfungselement (20) eine Wandaus-weitung (21-24) umfasst, die so geformt ist, dass sie zumindest einem Teil der Außenkonturen des Leitungselements (7) und der ersten, zweiten und dritten Druckabgriffsstutzen (8, 9, 10) entspricht, um eine Fluidabdichtung zwischen diesen zu gewährleisten.

3. Einrichtung nach Anspruch 2, **dadurch gekennzeichnet, dass** das erste Dämpfungselement (20) zwischen dem ersten und dem zweiten Gehäuseteil (1a, 1b) eingeschlossen ist.

4. Einrichtung nach einem der Ansprüche 2 oder 3, **dadurch gekennzeichnet, dass** die Wandausweitung (21-24) des ersten Dämpfungselements (20) einen bogenförmigen Abschnitt (21) von halbzylindrischer Form und mindestens drei Leitungsabschnitte (22, 23, 24) umfasst, die mit dem bogenförmigen Abschnitt (21) fest verbunden sind und in Fluidkommunikation mit dem Inneren des bogenförmigen Abschnitts (21) stehen.

5. Einrichtung nach einem der Ansprüche 2 bis 4, **dadurch gekennzeichnet, dass** die drei Leitungsabschnitte (22, 23, 24) um die ersten, zweiten und dritten Druckabgriffsstutzen (8, 9, 10) herum angebracht sind, wenn das Dämpfungselement (20) auf dem ersten Gehäuseteil (1a) angeordnet ist.

6. Einrichtung nach einem der Ansprüche 2 bis 5, **dadurch gekennzeichnet, dass** das erste Dämpfungselement (20) eine Gehäuseöffnung (25) umfasst, und das Mikrogebläse (4) einen in einem Gehäuse (13) angeordneten Elektromotor umfasst, wobei das Gehäuse (13) des Mikrogebläses (4) in der Gehäuseöffnung (25) des ersten Dämpfungselements (20) untergebracht ist.

7. Einrichtung nach einem der vorstehenden Ansprüche, **dadurch gekennzeichnet, dass** ein zweites Dämpfungselement (30) aus flexiblem Material, das einen internen Durchlass (31) umfasst, am Ausgang (5) des Mikrogebläses (4) angeordnet ist und an einer internen Trennwand (12) befestigt ist, die das erste Innenfach (2) und das zweite Innenfach (3) trennt, um eine Fluidabdichtung zwischen dem ersten Innenfach (2) und dem zweiten Innenfach (3) und eine Fluidkommunikation zwischen dem Ausgang (5) des Mikrogebläses (4) und dem zweiten Innenfach (3) zu gewährleisten.

8. Einrichtung nach Anspruch 7, **dadurch gekennzeichnet, dass** das zweite Dämpfungselement (30) zwischen der internen Trennwand (12) des ersten Gehäuseteils (1a) und dem ersten Dämpfungselement (20) angeordnet ist.

9. Einrichtung nach einem der Ansprüche 7 oder 8, **dadurch gekennzeichnet, dass** das erste und/oder zweite Dämpfungselement (20, 30) aus Elastomer oder thermoplastischem Elastomer besteht und/oder der erste und zweite Gehäuseteil (1a, 1b) aus Polymer besteht.

10. Einrichtung nach Anspruch 2, **dadurch gekennzeichnet, dass** der zweite Gehäuseteil (1b) mindestens drei Druckabgriffsöffnungen (42, 43, 44) umfasst, die mit den mindestens drei Leitungsabschnitten (22, 23, 24) des ersten Dämpfungselements (20) zusammenwirken.

11. Einrichtung nach Anspruch 2, **dadurch gekennzeichnet, dass** der zweite Gehäuseteil (1b) mehrere Drucksensoren (50, 51) mit Druckabgriffen (52, 53, 54) umfasst, die über mindestens drei Leitungsabschnitte (22, 23, 24) des ersten Dämpfungselements (20) an dem ersten, zweiten und dritten Druckabgriffsstutzen (8, 9, 10) des Leitungselements (7) des ersten Gehäuseteils (1a) fluidisch angeschlossen sind.

12. Einrichtung nach Anspruch 11, **dadurch gekennzeichnet, dass** die Drucksensoren (50, 51) von mindestens einer im zweiten Gehäuseteil (1b) angeordneten elektronischen Karte (55) getragen werden.

13. Einrichtung nach einem der Ansprüche 11 oder 12, **dadurch gekennzeichnet, dass**:
- es sich bei mindestens einem Drucksensor (50, 51) um einen Differenzdrucksensor (51) handelt, der an zwei Druckabgriffsstutzen (9, 10) der ersten, zweiten und dritten Druckabgriffsstutzen (8, 9, 10) des Leitungselements (7) angeschlossen ist, und
- ein Druckverlustelement (35) in dem Gasdurchlass (11) zwischen zwei Druckabgriffsstutzen (9, 10) der ersten, zweiten und dritten Druckabgriffsstutzen (8, 9, 10) des Leitungselements (7) angeordnet ist.

## Claims

1. Breathing apparatus comprising a case (1) containing a motorised micro-blower (4), **characterised in that**:
- the case (1) is formed from at least one first and one second portion of a case (1a, 1b) assembled to one another, the first case portion (1a) comprising a first internal compartment (2) and a second internal compartment (3),
- the micro-fan (4) is arranged in the first internal compartment (2) by being sandwiched between the first and second case portions (1a, 1b),
- the outlet (5) of the micro-fan (4) is in fluidic communication with the second internal compartment (3),
- the second internal compartment (3) comprises a gas outlet orifice (6) in fluidic communication with a duct element (7) integral with the first case portion (1a), and
- the duct element (7) comprises a gas passage (11) and at least one first, one second and one third pressure tap tubing (8, 9, 10) integral with the duct element (7) and in fluidic communication with the gas passage (11).

2. Apparatus according to the preceding claim, **characterised in that** a first damper element (20) made of flexible material is arranged on the first portion of case (1a), said first damper element (20) comprising a wall expansion (21-24) shaped to hug at least one portion of the outer contours of the duct element (7) and of the first, second and third pressure tap tubings (8, 9, 10) so as to ensure a fluidic seal between them.

3. Apparatus according to claim 2, **characterised in that** the first damper element (20) is sandwiched between the first and second case portions (1a, 1b).

4. Apparatus according to one of claims 2 or 3, **characterised in that** the wall expansion (21-24) of the first damper element (20) comprises an arched portion (21) of semi-cylindrical shape and at least three duct portions (22, 23, 24) integral with the arched portion (21) and in fluidic communication with the inside of said arched portion (21).

5. Apparatus according to one of claims 2 to 4, **characterised in that** the three duct portions (22, 23, 24) are press-fitted around first, second and third pressure tap tubings (8, 9, 10), when the damper element (20) is arranged on the first portion of case (1a).

6. Apparatus according to one of claims 2 to 5, **characterised in that** the first damper element (20) comprises a casing opening (25), and the micro-fan (4) comprises an electric motor arranged in a casing (13), said casing (13) of the micro-fan (4) being housed in the casing opening (25) of the first damper element (20).

7. Apparatus according to one of the preceding claims, **characterised in that** a second damper element (30) made of flexible material comprising an internal passage (31) is arranged at the outlet (5) of the micro-fan (4) and integral with an internal wall (12) that separates the first internal compartment (2) and the second internal compartment (3) so as to ensure a fluidic seal between the first internal compartment (2) and the second internal compartment (3) and a fluidic communication between the outlet (5) of the micro-fan (4) and the second internal compartment (3).

8. Apparatus according to claim 7, **characterised in that** the second damper element (30) is sandwiched between the internal wall (12) of the first portion of case (1a) and the first damper element (20).

9. Apparatus according to one of claims 7 or 8, **characterised in that** the first and/or second damper elements (20, 30) are made of elastomer or of elastomer thermoplastic, and/or the first and second case portions (1a, 1b) are made of polymer.

10. Apparatus according to claim 2, **characterised in that** the second case portion (1b) comprises at least three pressure tap orifices (42, 43, 44) that engage with the at least three duct portions (22, 23, 24) of the first damper element (20).

11. Apparatus according to claim 2, **characterised in that** the second case portion (1b) comprises several pressure sensors (50, 51) comprising pressure taps (52, 53, 54) fluidically connected to the first, second and third tubings (8, 9, 10) of the pressure tap of the duct element (7) of the first portion of case (1a) through at least three duct portions (22, 23, 24) of the first damper element (20).

12. Apparatus according to claim 11, **characterised in that** the pressure sensors (50, 51) are carried by at least one electronic card (55) arranged in the second case portion (1b).

13. Apparatus according to one of claims 11 or 12, **characterised in that**:
- at least one pressure sensor (50, 51) is a differential pressure sensor (51) connected to two pressure tap tubings (9, 10) of said first, second and third pressure tap tubings (8, 9, 10) of the duct element (7), and
- a load loss element (35) is arranged in the gas passage (11) between two pressure tap tubings (9, 10) of said first, second and third pressure tap tubings (8, 9, 10) of the duct element (7).
